# EUROPEAN PATENT APPLICATION

(11) **EP 4 800 391 A1**
(43) Date of publication of application: **02.09.2026**
(21) Application number: 25305263.3
(22) Date of filing: 27.02.2025
(51) Int. Cl.: G01N 33/15, G01N 35/00, G01N 35/10

(54) **FLOW-THROUGH FLUID MONITORING ASSEMBLY THAT REDUCES LOSSES IN A CALIBRATION MODE AND METHOD FOR CALIBRATING A SINGLE-USE SENSOR USING THE ASSEMBLY**

(71) Applicant: Sartorius Stedim FMT, 13400 Aubagne (FR)
(72) Inventor: GAY, Isabelle, 13400 AUBAGNE (FR)
(74) Representative: Plasseraud IP

(57) **Abstract**

The monitoring assembly (1) allows a biopharmaceutical fluid monitoring mode and calibration of a sensor (3), respectively, the monitoring by the sensor being performed within a conduit section, between an inlet valve (V1) and an outlet valve (V2) forming the ports for a calibration path. The assembly (1) integrates the sensor (3) laterally on the section, between two fluid connection ends, specifically between the valves (V1, V2) arranged directly on a conduit section rigid body (8) delimiting a lumen. Once the valves are selectively open and combined with closed switching parts (43, 44), circulation through the flow line of the monitoring mode is prevented, except through the lumen that becomes interposed: between calibration line (15) connected to a port (11) of the inlet valve, and a port (12) of the outlet valve serving to discharge a process fluid coming from calibration line (15) and specific for sensor calibration.

## Description

### Technical Field

This disclosure pertains to the field of inline monitoring of parameters in biopharmaceutical field, with use of measuring instruments in sterile conditions. More precisely, a fluid monitoring assembly with embedded sensor(s) is provided and a method using this assembly for calibrating a single-use sensor is also proposed.

### Background Art

Integration of measuring instruments, including probe devices that are calibratable, is known in the art and can take a variety of forms. Some probe devices are specifically designed for certain purposes. For example, pH sensor devices are designed for measuring the pH of a medium, with measurement of pH typically requiring calibration of the pH sensor device. More generally, disposable sensor devices can be configured to allow such calibration.

There is a clear trend in the biopharmaceutical industry to use single-use equipment in clinical trial manufacturing or even commercial manufacturing, both following so-called "current good manufacturing practices" regulations (cGMP). Most often such processes require different control loops for especially critical process parameters (CPP) in order to keep process and product quality within defined limits. For such control loops the usage of reliable sensors, possibly single-use sensors, as measuring devices is necessary. A great variety of such sensors are known for different physical or chemical properties such as pH, dissolved oxygen (DO), conductivity, pressure, flow, temperature, turbidity, viscosity etc.

Application of one or more sensor(s) in a cGMP environment for CCPs requires integrity and reliability of the sensor and its measurement value output over the whole process or batch duration to ensure the quality level over the full production time or production lot, respectively. For this purpose, a standard way on assessing the measurement capability of an individual sensor component is the pre-use and post-use calibration of a sensor in order to guarantee that the sensor as such was capable to measure within a certain accuracy range over the complete duration of its use. In case of a sensor that can be disconnected, e.g. in a stainless-steel flow line assembly, the sensor is calibrated outside the flow line or in a specific bypass arrangement. After calibration, the flow line is sanitized/ sterilized including the assembled sensor. The process is then run, knowing that the sensor was working in specification at the beginning of the process. After finalizing the process, e.g. the batch, the flow line is again sanitized/and deactivated by use of caustics such as 3-molar sodium hydroxide (NaOH) and rinsed with water for injection (WFI) afterwards. After that step, the sensor can be disassembled and calibrated again in comparison to a reference sensor or with a certified standard.

In case of the need or demand for a fully closed process, not allowing any disconnection, this workflow does not apply anymore. Especially the disassembly of the sensor from the single-use tube line assembly contradicts a closed system or in general a disposable approach.

As disclosed in document US 11112380, a way to prevent any disconnection is having a calibration medium initially contained in a cuvette area provided in the flow line of the measuring system. Such medium fills the cuvette to the extent that the sensor element is in contact with the medium and can therefore record a pH measured value of the medium. This can, therefore, be used to calibrate the measuring component that remains isolated from outside environment. Then the measuring system can be connected to the process lines via membrane connectors ensuring sterile connection.
However, in many applications, there is a need for allowing calibration after the flow line is constituted.

Document EP 4257667 A1 discloses a bioprocessing fluid sensor arrangement for sensing fluidic properties in a bioprocess fluid path with a sensor, configured for aseptically connecting the sensor with another fluid, generally called reference fluid, while separating the sensor from the bioprocess fluid, cleaning of the sensor. A reverse flow mode is also available. Two sets of three switches are required to have separation of the sensor from the bioprocess fluid to be monitored. The arrangement proposed in this document, provided with flexible hose sections to define the flow line equipped with the sensor, involves many plastic components used for the consumable parts (not in favor of sustainability), to ensure aseptic connections. The assembling of the different parts is quite fastidious.
Moreover, the bypass volume is leading to high amounts of calibration and rinsing fluids.

More generally, there is still room for improvement regarding constructions of fluid monitoring arrangements.

### Objects and Summary

For improving situation, embodiments of the invention provide a biopharmaceutical flowable product monitoring assembly, comprising:
- a segment of conduit comprising a wall defining a lumen through which the flowable biopharmaceutical product passes, the segment having a longitudinal axis and extending longitudinally between a first fluidic connection member for attachment to a flexible hose and a second fluidic connection member for attachment to another flexible hose;
- at least one sensor mount, integrally formed with the wall, comprising a sensor mount that can extend generally transverse with respect to the longitudinal axis, the sensor mount being typically annular and including an aperture (with the aperture adapted to define an inner surface extending through the sensor mount to the lumen);
- a sensor secured within the sensor mount, preferably removably, the sensor being an integrated sensor for measuring or detecting a physical or chemical property, possibly by measuring pH, at a measurement location in the lumen (i.e. in the monitoring flow line in normal operation of the bioprocess, to which the monitoring system can be associated), this location being also called first measurement location when several sensors are embedded/mounted on the wall;
- an inlet valve and an outlet valve, each arranged to be formed on a side of the wall;
- a longitudinal wall body, rigid and forming all or part of the wall, preferably shaped as a shell to delimit an interior volume included in the lumen, the longitudinal wall body comprising two hollow valve housings and allowing integration, in said assembly, of an inlet port included in the inlet valve and of an outlet port included in the outlet valve, the inlet port being configured for fluid connection with a calibration line;
wherein the at least one sensor mount is provided between two spaced passages through the wall that consist of:
- a first passage for radial fluid communication between an inlet port opening and the lumen, the inlet port opening being provided in the inlet valve and the first passage being closable by a first movable member of the inlet valve; and
- a second passage for radial fluid communication between the lumen and an outlet port opening provided in the outlet valve, the second passage being closable by a second movable member of the outlet valve with the outlet port opening provided beyond a closing part or plug of the second valve member that is movable in/inside the body, the closing part being preferably displaceable in a hollow (in a corresponding valve housing) that typically extends adjacent to the lumen,
wherein the inlet valve and the outlet valve, each adjacent to the wall, allow a line/calibration line, selectively bypassing the fluidic connections obtained at the respective first and second fluidic connection members, to be used in a sensor calibration mode.

According to preferred embodiments, the valves allow (respectively) the calibration line and a discharge line (having for instance a waste container) to be connected via the lumen, in a calibration mode with the passage released due to an open configuration of the inlet valve. The inlet valve may be longitudinally offset and adjacent to the first fluidic connection member, while the outlet valve may be longitudinally offset and adjacent to the second fluidic connection member. The outlet valve can release the second passage, at least when flushing a calibration process media and a rinsing fluid (WFI typically), in the calibration mode. The monitoring assembly can be used with the open state of the outlet valve, respectively with open state of the inlet valve, only when the lumen is selectively a part of the calibration path, e.g. with the two opposite hoses pinched or plugged to have a non-flowing configuration in the monitoring line.

Thanks to this arrangement, less waste is generated, with typically space needed for the set-up, knowing that two or possibly more sensors can be integrated via the mounting structure serving to delimit the lumen, and less plastic components used for the consumable. The fluidic connection members can advantageously be end members/connecting members that are axially opposite, along the longitudinal axis, with an interspace reduced to be inferior to 120 or 200 mm, lower than 70 or 80 mm in some options (with longitudinal distance between the passages serving for the calibration mode, corresponding to an extension length of the wall where a mounting structure is provided, being possibly lower than 50 or 60 mm for instance).

Measurements can be done in a first mode (monitoring mode) with the assembly forming part of a main flow path, while quality of some measures can be enhanced, by circulating an amount of fluid, possibly limited, through the assembly used as a bypass/short section in a calibration path in sterile conditions. The calibration is obtained using:
- the inlet port, forming inlet of the calibration bypass,
- and the outlet port, forming outlet of the calibration bypass.
Advantageously, the lumen (between the connecting end members) is used for at least one calibration workflow. This can be a single use tube line in some embodiments, with the sensor being a single-use sensor.

The assembly can advantageously integrates the sensor laterally on the conduit section, between two fluid connection ends and specifically between the valves arranged directly on the conduit section rigid body delimiting a lumen. Once the valves are selectively open and combined with closed switching parts, circulation through the product containing flow line parts of the monitoring mode is prevented, except through the lumen that becomes interposed:
- between calibration line connected to the port (inlet port) of the inlet valve,
- and the port (outlet port) of the outlet valve serving to discharge the process fluid coming from calibration line and specific for sensor calibration.

Of course circulation through the opposite fluidic connection members can be stopped, using suitable switching means, for instance a pair of clamps or any suitable switching parts, so that the flow-through measuring area equipped with the sensor can be only used with a calibration process fluid (distinct from the biopharmaceutical fluid to be monitored).
The wall can be entirely rigid, defining a tubular structure extending from a nozzle of the first fluidic connection member to a nozzle of the second fluidic connection member. The wall may be provided with an outer surface and two tubular outer shells protruding radially from the outer surface of the wall. The two tubular outer shells can protrude, each, in a direction distinct from a protruding direction of the mount. This may be a same protruding direction, with the inlet valve and the outlet valves (arranged parallel) being preferably of same length.

According to some embodiments, the valves can be compact and/or be provided with valve members that are arranged to limit path size between the plugs or closing parts of the valves. In options of the monitoring assembly, one or more of the following dispositions are provided:
- the inlet valve has a movable valve member delimiting at least one fluid channel of the inlet port and provided with an actuatable portion, preferably a rotating flange, that carries or includes an nozzle forming an end of the at least one fluid channel;
- in a valve open state, the nozzle of the inlet valve is opposite to another end of the at least one fluid channel, such another end opening in the lumen, possibly with the valve member more inserted in the valve housing (of the inlet valve) in the valve open state, as compared to a valve closed state.
- the outlet valve has a movable valve member delimiting a given fluid channel of the outlet port and provided with an actuatable portion, preferably a rotating flange, that carries or includes another nozzle forming an end of the given fluid channel.
- in a valve open state, the nozzle of the outlet valve is opposite to another end of the given fluid channel, such another end opening in the lumen, possibly with the valve member more inserted in the valve housing (of the outlet valve) in the valve open state, as compared to a valve closed state.
- one or two of the valves are plunger valves.
- at least one valve member may be provided with a plunger, shaped as a narrow shell that has an insertable part, movable perpendicular to the longitudinal axis, inside the corresponding valve housing/valve body.
- the two valve housings are valve bodies included in a plastic piece that is a hollow piece belonging to the wall and provided with a wide side opening closed by a mounting structure, which is a structure or piece including the at least one sensor mount.
- each plunger can carry a plug/closing part at a proximal end close to the lumen and/or is provided with a plug/closing part is adapted to be displaced inside the lumen, for instance to release a valve seat provided at a junction between the valve housing and a rigid duct of the wall delimiting the lumen.

With such embodiments for integration of the valve, when having a plunger structure for the valve member, the common volume involved for circulation of the biopharmaceutical fluid and for circulation of at least one process media (calibration fluid / rinsing solution such as WFI) can be greatly reduced inside the assembly.
The passages through the wall being provided between the two fluidic connection members, the assembly can be compact, typically with one or two sensors mounted adjacent along the longitudinal direction to fill the gap between the two passages that each allow radial fluid communication between the lumen and components of calibration path. For instance a filter, preferably a sterile/sterilized filter, of the calibration line can be mounted with an overlapping area of the filter with the inlet valve, while an aseptic connector carried by a waste container or bag can be directly connected to the outlet valve, also with an overlapping area (typically with the outlet port opening surrounded by a rigid end of the aseptic connector).

Embodiments of the disclosure provide a monitoring assembly having a pair of valves each having a valve body/housing guiding the valve member in a radial direction, with one or more of the following features provided:
- each valve amongst the inlet valve and the outlet valve comprises a valve housing/valve body delimited by a tubular wall section that is perpendicular to the longitudinal axis and opens into the lumen.
- each valve member is displaced radially inward to move from a closed position distal from the longitudinal axis to an open position proximal to the longitudinal axis, preferably with the valve member protruding inside the lumen.
- each valve member has a hose barb, a Luer connecting part (standard connection) or a similar nozzle, protruding from an actuatable portion.
- rotation of the actuatable portion (rotary action on the valve member) cause the displacement of the valve member, along radial direction.
- each valve housing is provided with a stop that engages a pin or latching member, included in the valve member, in the closed position of the valve member, so that removal of the valve member away from the valve housing is prevented.
- each valve housing includes a pair of two stops for engagement with two respective pins or latching members, included in the valve member, in the closed position of the valve member, so that removal of the valve member away from the valve housing is prevented.
- a stop or each stop is arranged adjacent to an external groove or slot guiding displacement of the valve member;
- each valve has a bayonet connection with an external groove or slot guiding displacement of the valve member, the stroke of the valve member being limited by two opposite stops forming ends of the external groove or slot.
- each valve has a bayonet connection with an external groove or slot guiding displacement of the valve member, the stroke of the valve member being limited: by a stop formed at an end of the external groove or slot, and by a flange portion arranged to abut onto annular rim delimiting a valve housing opening through which the valve member is inserted.
- the valve member comprises a tab, extending parallel to the valve housing and carrying the pin or latching member, the tab extending outside the valve housing with the pin or latching member protruding from the tab, toward the valve housing to be slidably mounted in a corresponding external groove or slot.
- the wall includes a rigid duct piece, constituting the body, each of the two valve housings being part of the rigid duct piece.
- for at least one valve, the actuatable portion of the valve member is arranged to be more distant, from the longitudinal axis, than an opening of the valve housing for insertion of the valve member.
- for the inlet valve and/or the outlet valve, the valve housing comprises a tapering portion, preferably adjacent to the lumen and/or providing an annular seating region for an annular sealing element carried by/included in the valve member.
- each valve housing is provided with an end detent member is engaged with the pin, relief or latching member, to prevent a reverse displacement of the valve member and keep the open position. The tab and/or the detent member can be resiliently deformable to allow a removable engagement or snap-action of the latching member.

The monitoring assembly can have a substantially constant cross section in the inner face (of tubular shape) delimiting the lumen for circulation of the biopharmaceutical product through the section of conduit, as measured at any cut plan perpendicular to the longitudinal axis. Besides, in closed state for the inlet valve and the outlet valve, each closing part of the valve members can extend flush with the internal face of the wall (wall of the section of conduit).

In preferred embodiments, one or two of the valves is/are actuatable by a rotary action, to displace the movable valve member from the open state to the closed state and vice versa. Each valve member can comprise one or more reliefs to also engage a slot or internal groove (of the valve housing) guiding displacement of the valve member. The valve housing may be provided with two slots, while the valve member can engage each slot:
- by a relief formed on the insertable part of the valve member, on the one end; and
- by a pin or latching element provided in the external part of the valve member, using a tab or similar overlapping element that is overlapping the valve housing.

A compact structure of the wall, including the two valve housings, may be obtained, for instance with the body of the wall only having two branches that consist of the valve housing of the inlet valve and the outlet valve, respectively. Besides, no flexible hose part is present in the section of conduit or between the section of conduit and the inlet opening coupled to a component, preferably a filter, of the calibration line, so that the assembling operation to obtain the monitoring assembly is simplified.
The inlet port in the inlet valve can feature a Luer lock connection for direct integration with the filter.

Optionally, for each valve, the valve member comprises a tubular structure and extends between a first end part forming an insert member inserted in the valve housing and a second end part, forming a fluid outer connecting end that opens, at an opening, along a direction perpendicular to the longitudinal axis. Typically, the outer connecting end of the valve member can be:
- chosen amongst a barbed nozzle, a Luer lock connecting part and bayonet connection part, or other kind of quick coupler;
- overlapped by a hose end or a component coupler provided in the calibration line.
Besides, in the valve, the elongated hollow channel can extends, straight or rectilinearly, between the opening at the outer fluid connecting end and a closed end that is:
- adjacent to a plugging part/closing part of the valve member;
- provided with at least one transverse passage directly communicating with the lumen in open state of the valve.

In each valve member, at least one sealing element can be provided to define an annular contact with inner surface of the valve housing. Two annular contacts can be obtained. The plugging or closing part may be provided with a first sealing element, for instance an O-ring or similar annular sealing element, possibly made of an elastomer.
In preferred options, the valve member is provided with a second sealing element, of annular shape and possibly surrounding a tubular section of the valve member.
For a given valve member, the distance between the two sealing elements is lower than a length of the rigid valve housing. Advantageously, a sensor of the monitoring assembly is configured to be recalibratable, possibly more than twice without contaminating any content present in the hoses coupled to the fluidic connection members (hoses of the main/first flow path involved for the monitoring mode). Arrangement with two sealing elements (or more) for the two valve members allows calibration process media to be better separated from the biopharmaceutical fluid, for instance by being retained (at the closure of the valves) in an intermediate area between the two sealing elements, which can be an annular/peripheral area communicating.

In the monitoring assembly, at least one valve amongst the inlet valve and the outlet valve is actuatable by a rotating action, around a valve axis, preferably by using a bayonet connection for securing the valve member, which includes an elongated hollow channel, to the valve housing. The valve housing is typically integrally formed of molded plastic material with a rigid piece of the section of conduit.
The valve housing includes a bayonet mount as a fitting for a bayonet connection, the bayonet mount being provided with two groove portions adapted to cooperate with complementary fixation reliefs or pins formed in the tabs that are in an overlapping configuration relative to the valve housing. When the valve member has two sealing elements, each of them can be inserted, inside the valve housing, farther than position of the two groove portions.

When having the valves provided with valve members that are movable along a radial direction to reach the open state, one or more of the following dispositions can be provided:
- each valve amongst the inlet valve and the outlet valve comprises a movable valve member having an extension direction transverse to the lumen (i.e. transverse to the longitudinal axis), the valve member being typically longer that wide.
- a first sealing element a second sealing element, spaced from the first sealing element along length direction of the valve member, are provided on the movable valve member.
- the first sealing element is configured to be in sealing contact on a seating region formed by the section of conduit to close the valve.
- the second sealing element, having an annular shape, is adapted to follow displacement of the movable valve member and can separate an interior space of the valve from an outside of the monitoring assembly.

Typically, the first sealing element and the second sealing element can share a common central axis and/or are distributed, with a spacing, along the extension direction of the movable valve member.

According to embodiments of the monitoring assembly, one or two sensors can be integrated by passing through the thickness of the section of conduit. Typically, the section of conduit, forming a mounting structure so that each sensor mount is an integral part of the section of conduit, can be provided with one or more of the following features:
- the sensor has an elongated body terminating at one end thereof in a sensing member.
- the elongated body has a flange (on a portion thereof) and a cover plate that constitutes or rests on the sensor mount when secured to the elongated body.
- the wall consists of a plastic housing having an enlarged central part to form the at least one sensor mount, while also delimiting a centralized fluid path segment common to the two modes allowed by the monitoring assembly (monitoring mode and calibration mode with the valves open).
- the centralized fluid path segment can have substantially same cross section as the two end segments of the section of conduit provided each with a fluidic connection member.
- the enlarged central part is enlarged by a mounting structure, possibly including a cover plate or similar part that is made separate from the centralized fluid path segment.
- the plastic housing combines four rigid couplers for fluidic connection, two of the couplers being offset relative to a main path and included in the valves to allow direct connection with functional components of a calibration fluid path (with a quick connection allowed by each coupler formed as an end member in the valve).
- the two other couplers can be the fluidic connection members, possibly each having at least on barb and forming a nozzle.

In preferred embodiments, more than one sensor is integrated in the monitoring assembly. For instance, the monitoring assembly can comprise:
- two sensor mounts, so that the above presented sensor mount is a first sensor mount and the sensor is a first sensor provided with a first sensing member configured to be in contact with fluid - forming the biopharmaceutical product - passing through the lumen (in a monitoring mode),
- a direct integration in the section of conduit of the second sensor mount, which is integrally formed with the wall of the segment of conduit and extends generally transverse with respect to the longitudinal axis of the segment of conduit.
- an aperture, included in the second sensor mount, defining an inner surface extending through the second sensor mount to the lumen of the segment of conduit.
- a second sensor, which is secured, preferably removably, within the second sensor mount;

Typically, the second sensor is provided with a second sensing member configured to be in contact with the fluid passing through the lumen, so that the second sensor is another integrated sensor for measuring or detecting a physical or chemical property (distinct property preferably, i.e. different from the one measure by the first sensor) at another measurement location in the lumen.
In some options, the first sensor mount and the second sensor mount are formed on a cover plate or on common flange that is flat and preferably arranged as a cover plate, which seals a side access opening of the body. The side access opening is delimited by an annular rim and opens radially from the body. A welding can be performed to seal the cover plate that remains in annular sealing contact with the annular rim.

In some embodiments, the monitoring assembly may be formed as a single-use assembly, either with the sensor(s) removable from the corresponding sensor mount(s), or with each sensor disposable with the monitoring assembly. Additionally or alternatively, the section of conduit has two ends consisting of hose bar connectors, whereby the monitoring assembly is provided with a first hose barb connector and a second hose barb connector that are:
- inseparable, preferably made of same material in a same molded piece; and/or
- facing opposite directions, while extending parallel to the longitudinal axis, the longitudinal axis preferably being a symmetry axis for the first hose barb connector and the second hose barb connector.
It is understood that these hose barb connectors can constitute the first fluidic connection member and the second fluidic connection member.

Optionally, the wall is assembled from:
- a first piece forming the body, including the first hose barb connector and the second hose barb connector, and
- a second piece that is a cover plate having a top surface and including the at least one sensor mount formed on the top surface side.
For instance, the wall may be provided with two opposite side recesses to form a handle part facilitating grasping of the monitoring assembly underneath the cover plate.

The following structural features of the section of conduit can be provided, individually or in combination:
- each hose barb connector is provided with a peripheral flange or stopping portion suitable to stop an overlapping hose end of the hose coupled to this connector.
- length of the non-overlapped area of the section of conduit (area not dedicated to hose connection), as measured between the two flanges or stopping portions, can be lower than 120 mm or 200 mm, possibly inferior to 85 mm [TBC].
- the first passage and the second passage are typically the sole two transverse passages provided through the wall W, beyond the at least one mount/mounting structure (only two radial passages, except the sensor mount aperture(s)).

More generally, the structure of the monitoring assembly remains compact, simple, for instance without any movable part(s) extending inside the lumen in the monitoring mode, between the hose barb connectors.

The monitoring assembly can be part of monitoring and calibration assembly, including a calibration line provided with the waste container, the switching parts for separating the lumen from the hose sections used in the monitoring mode with circulation along a main/first flow path. For instance, the monitoring assembly typically comprises:
- the calibration line and two switching parts that are combinable with the inlet valve and the outlet valve to switch between a monitoring mode and a calibration mode, the monitoring assembly being adapted to be set, selectively in two configurations. These configurations include:
- a first configuration to define a first flow path, in which the two switching parts are open to allow a continuous circulation along a rectilinear main channel formed all a whole length of the section of conduit, while the inlet port for communication with the calibration line and the outlet port remain closed by closing the inlet valve and the outlet valve, whereby the monitoring mode is obtained; and
- a second configuration to define a second flow path, in which the two switching parts are closed while the inlet valve and the outlet valve are each in an open state to have a flow coming from the calibration line circulating successively through the inlet port, through the lumen and through the outlet port.

The calibration line, when included in the monitoring assembly, can comprise reference means, for instance a reference solution (typically pumped in the calibration line) and a filter to allow a process media, prepared in advance, to be supplied and filtered in the calibration line upstream the lumen, so that the flow coming from the calibration line is a flow of the filtered process media. This flowable media may include or consist in the reference solution. WFI may also circulate in the calibration line, for instance at transition steps preceding and/or following a calibration step performed with the monitoring assembly set in the monitoring mode.
It is understood that the monitoring assembly can be a two-valve system (without complex three-way valve(s)), thus being of simple construction with the body and/or the section of conduit deprived from any movable parts, regulating device or electronical/electrical components/plugs. The two valves can be used as one-way valves, for instance using synchronizing means to ensure the outlet valve is open when the inlet valve is open for the feeding with the process calibration media (coming from the calibration line).

According to another aspect, embodiments of the disclosure provide a method for calibrating a sensor in a measuring area where a monitoring is also performed for a given biopharmaceutical product, wherein the fluid monitoring assembly as above presented is provided (interposed between two flexibles hoses of a monitoring line for instance) so that the measuring area is located in an interior volume of the segment of conduit, between the inlet valve and outlet valve on the one hand, and between the first fluidic connection member the second fluidic connection member on the other hand,
wherein the method comprises, in a preparation phase:
- actuating switching parts to have the lumen isolated from other portions of a main flow path where the given biopharmaceutical product can flow, the lumen being delimited by an inner face of the segment that is constructed as a rigid part;
- functionally connecting a calibration line to the inlet port by opening the inlet valve (i.e. allow fluid communication through the inlet valve);
- functionally connecting a discharge line to the outlet port by opening the outlet valve (i.e. allow fluid communication through the outlet valve);
and wherein the method then comprises a calibration of the sensor mounted between the inlet valve and the outlet valve, which is performed with flowing of a calibration process media in a circulation phase, the circulation phase comprising:
- feeding the measuring area in the calibration process media via the inlet valve open, while the sensor arranged in the measuring area is in contact with the calibration process media;
- allowing the calibration process media to be discharged from the lumen to the discharge line via the outlet port to have a preferably discontinuous circulation of the calibration process media through the measuring area; and
- retrieving a measured value detected by the sensor.

Discontinuous circulation of the calibration process media can be caused by a closing of the outlet valve and a closing of the inlet valve. More generally, any fluid that is supplied through the calibration line (first line section upstream the inlet valve) can be moved either by a calibration pump provided in this calibration line or by a main pump involved in the monitoring mode (in case the main pump is located downstream of a switching part that can close circulation through the second fluidic connection member).

Accordingly, the assembling of the two paths is simple and compatible with an efficient monitoring operation consolidated by calibration step(s), while minimizing the flexible parts and the complex connections/junctions. Since the lumen can be a short path with no significant other channels in the calibration mode, a minimized volume of calibration and rinsing fluids can be obtained (typically with at least 10 or 20 mL fluid path reduction even for relatively low cross sections of the lumen). The section of conduit may be a graspable flow-through sensing body, without any T connectors coupled to three flexible hoses in particular.
Such sensing body can easily be controlled, manually, at actuations portions driving the valve members, while pinch valves or similar devices of simple construction can form switching parts, closable when calibration mode must be set. In some options,

In the method, more than one calibration can be performed. For instance, a first calibration can correspond to a phase performed before a first monitoring operation. A further calibration for controlling accuracy of the measures can occur, being possibly repeated, after such monitoring operation.
Typically, the discharge line includes a waste container or bag, preferably with a standard coupler included in the bag directly connectable to the outlet port included in the outlet valve.

In some options, the method comprises isolating the monitoring assembly from the line to be monitored, by closing two opposite access sections, typically corresponding to a monitoring assembly inlet and a monitoring assembly outlet, possibly formed in the connection members or adjacent thereto.

The method can comprise positioning the valve members (respectively distribute in the inlet valve and the outlet valve) in an inserted position/maximally inserted position, in which the closing part of each valve member is defining a face, distal from an actuation portion of the valve member, which is a face:
- flush with the inner face of the segment;
   and/or
- arranged to be closer to the longitudinal axis than an outer side of a cover plate, extending parallel to the longitudinal axis, when the monitoring assembly is provided with a cover plate secured to the body and forming all or part of the at least sensor mount.

The switching means/parts are manually operable clamps in some options, for instance clamps that are mode of two halves that allow quick positioning around a pinching area of the hose, which is adjacent to the corresponding end (axial end) of the assembly. The circulation of the calibration process media can be discontinuous to minimize amount of the media to be used in any measuring step of the calibration mode. In some variants, a continuous circulation can be provided, with the valves open thanks to the preparation phase. Such continuous circulation may be performed only for a rinsing phase in some options, while the calibration phase can be operated with the outlet valve open only after injection a rinsing solution via the inlet valve.

The switching parts can be distributed to close the first fluidic connection member or a hose coupled thereto, and the second fluidic connection member or a hose coupled thereto, respectively. The method may comprise, in the preparation phase:
- assembling a calibration line provided with a pump and a filter, the calibration line being further provided with at last one amongst a reference sensor, a reference source or a reference standard solution related to a physical or chemical property to be measured or detected by the sensor.
Additionally or alternatively, the calibration process media can be filtered in the calibration line before reaching the measuring area, during the feeding in the calibration process media, the feeding preferably using a pump provided upstream the filter. The (preliminary) assembling of the calibration line can be performed before operational use of the assembly, typically before any actuation of the valves for using the assembly in some options.

In some options of the method, the following dispositions are provided:
- each sensing member can extend through the lumen in some options.
- the sensor, which may be single-use sensor, is a first sensor of the monitoring assembly, while a second sensor is mounted on and secured to the segment of conduit that carries the first sensor, so that the first sensor and the second sensor are both mounted (on a same mounting structure, typically covering a side wall opening) with a sensing member located within said measuring area, between the inlet valve and the outlet valve.
- the monitoring is performed by using the first sensor and the second sensor simultaneously.
- the monitoring is performed after at least one calibration performed for the first sensor, with the given biopharmaceutical product flowing straight (along a rectilinear general direction) through the first fluidic connection member, the lumen and the second fluidic connection member.
- the inlet valve and the outlet valve are spaced from a longitudinal distance, which may be inferior or equal to 70 or 80 mm.
- the inlet valve and the outlet valve have each a rigid valve body formed integrally with the segment of conduit.
- the segment of conduit includes a single piece serving for a coupling with two hoses (of the monitoring path) and a guiding of two valve members (preferably parallel valve members movable perpendicular to the longitudinal axis).
- in the calibration mode, after injection of the calibration process media/solution in the lumen, the inlet valve can be closed when closing the outlet valve, so that no additional calibration process media is fed from the first inlet port into the measuring area.

The method can comprise a cleaning of the measuring area, by a cleaning liquid forming the rinsing solution, preferably water (WFI preferably), which is injected in the calibration line upstream the filter. Optionally, the sensor can be a single-use sensor, with a step of disposing the sensor with the section of conduit after use (after disconnection of the two flexible hoses for instance).

The method may comprise, before and/or after the monitoring:
- placing a sensing member of the sensor in a flow of a rinsing solution distinct from the calibration process media, the rinsing solution flowing through the measuring area by circulating through the inlet valve and being discharged via the outlet valve.
When the sensor is a calibratable pH sensor, the corresponding sensor mount is configured to define a sensor mount central axis (for the pH sensor), which intersects the longitudinal axis, perpendicularly. This sensor mount may also be perpendicular to direction of displacement of each valve member provided in the inlet valve and outlet valve.

In embodiments of the method, calibration of the pH sensor is carried out twice or more than twice, with the outlet port having a hose barb engaged with a hose or female connecting member, which communicates with an interior volume of a waste container or bag.
Optionally, the monitoring assembly is operating with the inlet valve and the outlet valve open simultaneously during each calibration, while the monitoring assembly is maintained in a sealed and sterile configuration, without any mechanical disconnection at the first fluidic connection member and at the second fluidic connection member.
Another sensor, mounted parallel to the pH sensor, can be a conductivity sensor.

In some embodiments, the discharge line is either only provided with a waste container or bag, or also provided with functional component(s), for instance a reference sensor so that the discharge line is forming a complementary calibration line completing the main calibration line coupled to the inlet port.
The inlet port can have a hose barb engaged with a female connecting member of a filter housing of an in-line filter (sterile filter), which is a filter mounted on the calibration line, preferably downstream a reference sensor and/or a reference source.

In the method, the sensor can be of the type calibratable at least twice. It allows to use n times, n being an integer of 2 or more, the sensor (including a pH probe device). It is easy to operate since the operator can, successively. More generally, it is understood that multi- points calibration of the sensor can be realized, efficiently, when such sensor is integrated as a part of a sterile single-use monitoring assembly having the two valves integrated adjacent to the lumen.

### Brief Description of Drawings

Other features, details and advantages will be shown in the following detailed description and on the figures, on which:
Fig. 1 illustrates a fluid monitoring assembly with integration of sensors, in a calibration mode, with the lumen inside the assembly wall isolated from the main flow path by a pair of clamps each configured in a closing state.
Fig. 2 is a cut view of a fluid monitoring assembly that is short, to illustrate a half of the monitoring assembly and integration of two sensors, having each a sensing portion arranged to be in contact with fluid passing in the lumen.
Fig. 3 is a schematic setup with the assembly combined with an exemplary calibration path provided with a set of liquid compositions.
Fig. 4 is longitudinal cut view, perpendicular to a sensor extension direction, showing an embodiment of a plastic housing having an enlarged central part to form at least one sensor mount and further combining four rigid couplers for fluidic connection, two of the couplers being separable from a main path and included in valves to allow direct connection with functional components of calibration fluid path.
Fig. 5A is a perspective view of an example of a valve member of a rotating valve, that allows for compact integration of the corresponding valve in the assembly, here the outlet valve for instance.
Fig. 5B is a perspective view of an example of a valve that includes a rotating valve member similar to the one shown in Fig. 5A, but with a different connecting nozzle.
Fig. 6A shows, by a cut view, a structure of a valve, here the inlet valve coupled to a sterile filter by a Luer lock connection, in a variant of a rotating valve integrated in the assembly to be adjacent to the lumen,.
Fig. 6B shows an exemplary structure of a rotative valve, similar to embodiment Fig. 6A with a radially protruding geometry, but with a hose barb nozzle forming the end coupler.
Fig. 7 is a flowchart illustrating steps of method using a monitoring assembly to obtain measured value(s) detected by a calibratable sensor mounted to have a sensing member directly in the single central flowing area (lumen) used both in calibration mode and in monitoring mode.
Fig. 8 is a flowchart schematically illustrating an embodiment for applying a calibration mode and then switching to a monitoring mode with a rectilinear flow passing through the measuring area, possibly with other calibration operation performed by changing the liquid/fluid flowing along the sensor(s) equipping the section of conduit of the assembly.
Fig. 9 illustrates a detail of a handle part provided with recesses and formed in an intermediate/central segment of the section of conduit in an exemplary embodiment, to allow a grasping of the monitoring assembly between the two valve bodies.

### Description of Embodiments

In the following, the wording "in monitoring mode" relates to an action performed with the assembly connected to a biopharmaceutical loop/circuit or a bioreactor, working in an in-line operation and the flow is generally called "main flow" in such case. The wording "in calibration mode" relates in contrast to a calibration procedure with a bypass configuration actuated, when the in-line operation is interrupted thanks to clamping or switching devices.
In the following, the words "outward", "inward" and their derivates are combined with "radially" and "longitudinally". With "radially", the point of reference is the longitudinal axis of the segment of conduit with "outward" and its derivates meaning "away from the longitudinal axis" and "inward" and its derivates meaning "towards the longitudinal axis". The term "top" is used to designated an extension direction of sensor(s) matching with illustrated embodiments, with knowledge a "top surface" designates an external surface orientated on a specific side, facing away from the longitudinal axis.

Detailed description of several embodiments of the invention is provided below, accompanied with examples and with reference to the drawings.
In the various figures, the same references are used to designate identical or similar elements. Length is to be understood in a usual way for the skilled person, following the longitudinal axis A as described below.

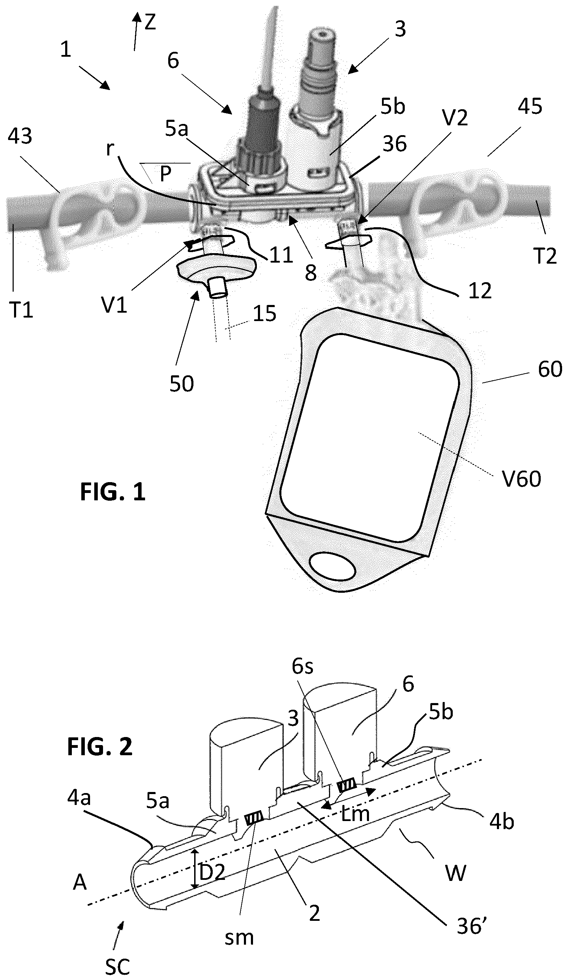

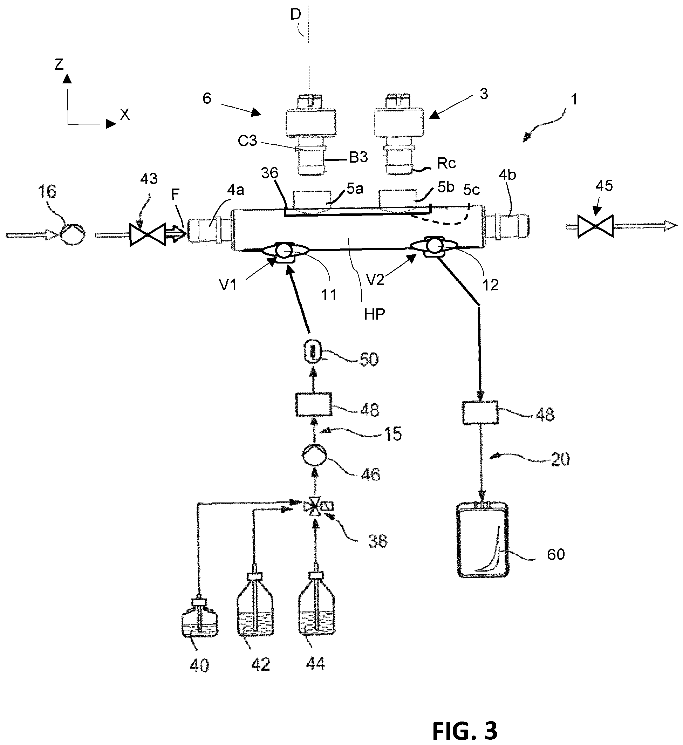

In one embodiment, the disclosure is described for a typical bioprocessing system, where a flowable biopharmaceutical product F (Fig. 3) is monitored through a line equipped with the monitoring assembly 1 such as illustrated in Figs 1 and 3-4. In use, the monitoring assembly 1 is part of a fluid path, i.e. an arrangement for conveying a fluid. The fluid path typically comprises one or more hoses T1, T2 or conduits (preferably flexible conduits) and a rigid section of conduit SC provided with a conduit connecting fluid inlet and a conduit connecting fluid outlet. The inlet and the outlet of the section of conduit SC can be part of a same block. In preferred embodiments, as illustrated in Figs 1-2 for instance, the conduit inlet is provided in a first fluidic connection member 4a, which is suitable for attachment to a flexible hose T1, while the conduit outlet is provided in a second fluidic connection member 4b suitable for attachment to another flexible hose T2.

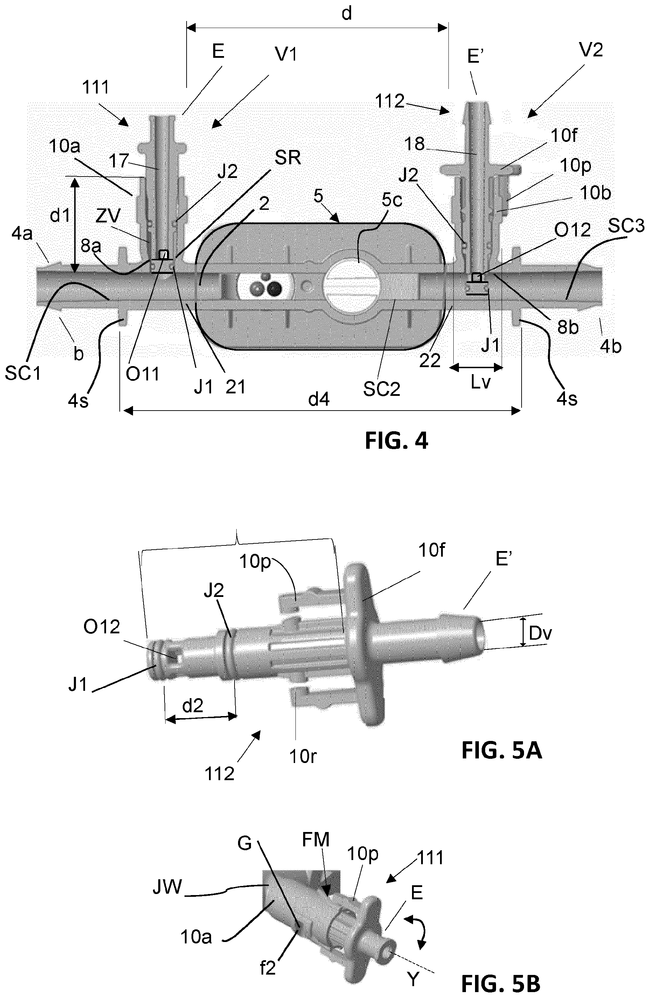

Referring to Fig. 1, two clamps forming a pair of switching parts 43, 45 are provided on the hoses T1, T2, respectively, to ensure that the section of conduit SC can be isolated, no more being in fluid communication with other components that are included in the entire fluid path where a bioreactor or similar bioprocess components are present. In open state of the clamps, the biopharmaceutical product F can flow inside the conduit section SC that integrates one or more sensors 3, 6 for a monitoring: this is monitoring mode. When a sensor has to be calibrated, prior to the monitoring or at a short step with interruption of the normal flow, pinched state is actuated, with the clamps thus ensuring closing of each of the hose lumens. More generally, any switching part can be provided, around a hose section adjacent to an end of the section of conduit SC, or being integrated as closing actuator in a nozzle or coupler forming such end. The section of conduit SC comprises two branches (each corresponding to branching JW), included in a common piece, forming a main piece of the section of conduit. The two branches, having a branching JW (Fig. 5B) made rigid in plastic material of the main piece, serve for a calibration mode obtained when switching off the monitoring mode, using two passages 8a, 8b of a calibration path.

Referring to Figs 1-2, the section of conduit SC can be constructed as a tubular rigid component, typically graspable by one hand. The members 4a and 4b form the tube ends of this rigid component, typically molded of plastic material (thermoplastic for instance), free of metal and compatible for contact with any biopharmaceutical fluid that can flow from a bioreactor or any path or circuit in a bioprocess. The section of conduit SC includes a wall W defining a lumen 2, through which the flowable biopharmaceutical product F passes in a monitoring mode. The wall W extends tubular around the longitudinal axis A of the segment of conduit SC. This longitudinal axis A may be a central axis common for the fluidic connection members 4a, 4b. These connection members 4a, 4b can be hose barb connectors and/or can form nozzles, in order to constitute the assembly inlet port and the assembly outlet port, allowing circulation of the product F in a monitoring mode. While a barb b or similar relief is shown in Figs 2, 3 and 4, more reliefs/barbs can used in some variants.

The wall W, typically provided with a hollow body 8 and a mounting structure 5 receiving at least one sensor 3, 6, can be a single wall component to delimit a single interior volume with a lumen 2. The mounting structure 5 is adapted for integration of each sensor 3, 6 between the two rigid branchings JW. The wall W typically extends axially along the longitudinal axis A, without containing any channel/secondary path running in parallel to the lumen 2 to join the hoses T1, T2 or the connection members 4a, 4b. A substantially constant inner diameter D2 can be defined in the section of conduit SC, which is a diameter of the lumen 2.
The plastic material of the section of conduit SC can combine chemical resistance and sterilizability. Non-limiting examples are: polypropylene (PP), polyethylene (PE), polyvinyl chloride (PVC), and polycarbonate (PC) are typically used due to their, and compliance with industry regulations.

### Exemplary mounting structure for integration of sensor(s)

The section of conduit SC can be provided with at least one sensor mount, integrally formed with the wall W. More generally a part of the wall W may be a mounting structure 5 with one or two mounts 5a, 5b (or more). In non-limiting example of Figs 1 and 2, the mounting structure 5 can be flat or under form of a plate, extending parallel to the longitudinal axis A. The mounting structure 5 can be designed to have each sensor 3, 6 provided on a same side of the section of conduit SC, which facilitates a grasping of the monitoring assembly 1.
A sensor mount 5a, 5b can be arranged to form an insertion passage, by extending generally transverse with respect to the longitudinal axis A of the segment of conduit SC. In the mounting structure 5, each sensor mount 5a, 5b can have an annular section and include an aperture 5c opening radially outwards. The aperture 5c typically defines an inner surface extending through the sensor mount to the lumen 2 or an interior space interposed between the mounting structure 5 and the lumen 2 and adapted to receive each fluid passing through the lumen 2.

To provide a compact mounting structure 5, this structure can be selectively provided on a middle segment SC2, between two side junctions with respective inlet and outlet valves (valves for a calibration mode). The ends 21, 22 of the middle segment SC2 can keep a same inner diameter (optionally with the diameter of the lumen 2 kept constant) and directly join the hose barb connectors forming the members 4a, 4b. The branching JW is interposed between a hose stop 4s (formed as a collar for instance) of the hose barb connector and axial ends of the mounting structure 5 that is not extending beyond the ends 21, 21 of the middle segment SC2.

In non-limiting embodiment of Figs 1 and 4 or in some similar constructions, spacing between the branching JW and the stops 4s, respectively between the branching JW and the mounting structure 5 can be small, for instance lower than 4 or 5 mm.
The connectors are the complementary segments SC1, SC3 of the middle segment SC2 in the section of conduit SC in exemplary embodiments.
Accordingly, the middle segment SC2 is a segment carrying or including the mounting structure 5, while also guiding the flow axially (due to a single/rectilinear extension of an inner face of the middle segment, without bent part), along the longitudinal axis A.

The middle segment SC2 is an intermediate tubular segment formed between the two end segments SC1, SC3 of the section of conduit SC, as apparent for instance from Fig. 4. The middle segment SC2, axially interposed between the junctions forming radial passages 8a, 8b (i.e. between the two branches), can have substantially same cross section as the cross section provided in each of the fluidic connection members 4a, 4b. This cross section of the middle section SC can thus be equal to cross section provided in each of the segments SC1, SC3. The two branches, preferably parallel, provide junctions at a same angular sector.

Referring to Figs 1 and 4, it can be seen that the two branches are part of a pair of valves V1, V2 arranged to close the passages 8a, 8b in the monitoring mode, possibly without modifying the cross section in the segments SC1, SC3 that joint ends of the middle segment SC2. Longitudinal extent Lv of each branching JW, here to provide each branch under form of a valve body 10a, 10b (shaped as a tubular valve housing), can be short to be not only lower than length extension of the mounting structure 5 but also lower than width or longitudinal extension Lm of the mount of each sensor mount 5a, 5b. The extent Lv, which is longitudinal increase of the wall W (along the longitudinal axis A) due to the inlet valve V1 or the outlet valve V2, may be selectively limited so that the two following relationships are satisfied: 2 Lv < d and Lv ≤ 2 D2
where d is the distance between the passages 8a, 8b.

Middle part of the lumen 2 (along the circulation length of the lumen) is thus provided inside the middle segment SC2, which can extend between two valve bodies 10a, 10b. Each valve body/housing 10a, 10b can extend/protrude radially outward from an elongated external face of the wall W, to define a T junction at respective radial openings/passages 8a, 8b formed in the elongated tube part. The distance d between the passages 8a, 8b for radial fluid communication with a calibration inlet port opening and a calibration port opening can be minimized and kept below 90 mm, for instance between 24 and 70 or 80 mm. Two sensor mounts 5a, 5b, having an interspace can be integrated in this short middle segment SC2. More generally, the distance d may be comprised between 20 to 200 mm, for example between 50 to 100 mm.

The mounting structure 5 can cover a side opening of the body 8, which has an opening plane P parallel to the longitudinal axis A. The wall W, provided with such mounting structure 5, has a locally enlarged central part allowing the at least one sensor mount to be formed peripherally on the wall W, while the central segment SC2 that carries the frame forming the side opening is also delimiting a centralized fluid path portion/segment. Such path portion is common to the two modes allowed by the monitoring assembly 1: a monitoring mode without using the passages 8a, 8b and a calibration mode with the valves open to selectively have circulation through the passages 8a, 8b.
The opening plane P, perpendicular to Z direction/extension of the probe(s) of each sensor (3, 6), can extend above the lumen 2 or flush with a top side delimiting the lumen 2. The fixation rim r, forming a fixation face orientated upwardly in Fig. 1 (Z direction being an up direction) may be rectangular, with two elongated sides parallel to the longitudinal axis A and two short transverse sides. The transverse sides may be spaced from a distance not exceeding 100 or 200 mm, possibly not exceeding 80 or 90 mm (possibly not exceeding three times the average outer diameter of the rigid wall W). The Mounting structure 5 may be entirely interposed, along longitudinal direction, between the valves V1 and V2 as in the case of Fig. 4 for instance.

Referring to embodiments of Figs. 1-4, two sensors 3, 6 can be provided on a same side of the section of conduit SC, with each a perpendicular extension to extend across the wall W at the middle segment SC2, with a spacing relative to the passages 8a, 8b. More generally, one or two sensors 3, 6, having each an elongated body B3, may be arranged on that side. They may terminate at one end thereof in a sensing member sm, possibly inside the lumen 2. A rigid fixture is established for each sensor. One or more of the sensors 3, 6 may be irremovably mounted on the section of conduit SC, for instance when having the section of conduit SC configured to be disposed after use. Alternatively, each sensor 3, 6 can be removably mounted on the section of conduit SC, thanks to a sensor mount 5a, 5b.
Fig. 3 shows that the body B3 can have a coupling male end, an annular bead Rc on an outer surface thereof or similar annular end structure, allowing a secure fixation, preferably with one or more sealing contacts established against the inner face of the corresponding sensor mount 5a, 5b. Additionally or alternatively, the sensor body B3 may have a collar or flange C3 on a portion thereof that rests on or within the sensor mount 5a,5b when secured within the sensor mount.

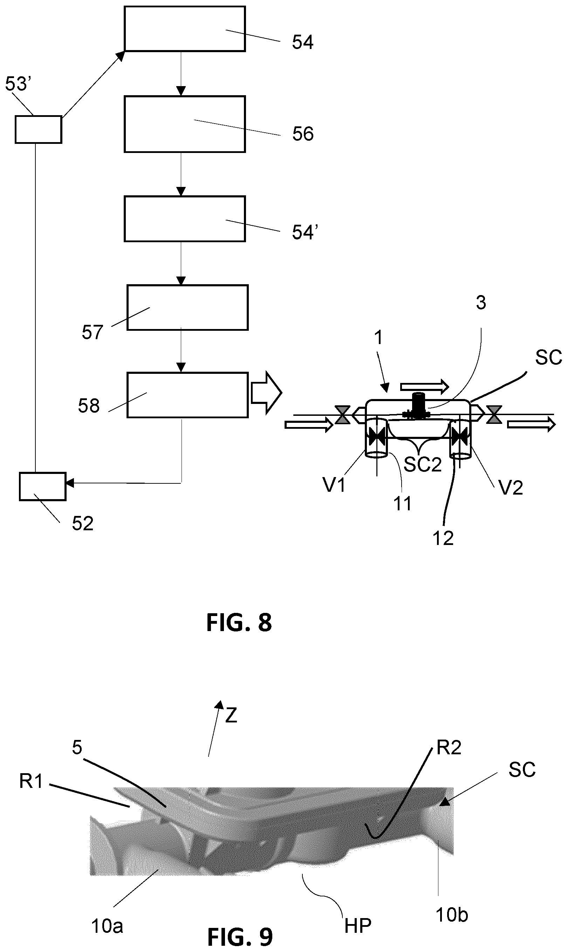

In some options for facilitating the assembling operations and handling of the assembly 1, the opposite side in the of the section of conduit SC (away from the sensor(s)) is comparatively narrower and/or provides an access to a gripping portion. More generally, a gripping portion can be here included in a middle fluid path segment SC2, forming a middle part of the section of conduit SC, as considered along the longitudinal axis A. In other words, as schematically illustrated in Fig. 3 when considering a top direction Z, which corresponds to a sensor extension perpendicular to the longitudinal axis A, the monitoring assembly can have a handle part HP. Here, the handle part HP extends along direction X of length of the section of conduit SC, and has a lower part (opposite to the sensor 3, 6) without any significant relief or bulge radially protruding outward from an external faces of the wall W, such that the section of conduit SC2 can include, as reflected in example of Fig. 9:
- at least one first recess R1, adjacent to a first side of the mounting structure 5 and provided, along Z direction, at same level as the lumen 2; and
- at least one second recess R2, adjacent to a second side of the mounting structure 5 (opposite from the first side) and provided, along Z direction, at same level as the lumen 2.

The mounting structure 5 can be obtained by fixing a cover plate 36 to an annular frame or flange, having a fixation rim r, typically of annular shape as in example shown in Fig. 1. When the elongated body 8 of the section of conduit SC includes a flange or fixation rim r (on a portion thereof, here typically on the middle segment SC2), the cover plate 36 can extend parallel to the longitudinal axis 1, using a fixation face secured on the fixation rim r. The sensor mount 5a, 5b, when secured to the rim r or similar fixation region, can ensure each sensor 3, 6 is fixedly inserted through the section of conduit SC when secured within the corresponding sensor mount 5a, 5b. An enlarged geometry of the cover plate 36, as compared to transverse size of the wall W at the opposite from the sensor(s), with a relatively wide side opening thus being created/delimited by the fixation rim r.

Alternatively, as in the non-limiting example of Fig. 2 for instance, the mounting structure 5 can include a narrow flat part 36' that possibly includes more than one sensor mount 5a, 5b. The narrow flat part 36' is here provided in an increased thickness portion of the wall W, as reflected in Fig. 2, with at least one sensor 3, 6 possibly screwed, snap fit or inserted in the sensor mount 5a, 5b to be secured in a sealed manner/with an annular sealing contact within the aperture of the sensor mount 5a, 5b. The lumen 2 can be of constant inner diameter D2, with each sensing member sm flush or adjacent to an inner face of the wall W.
Each aperture 5c may be relatively wide, as reflected for instance in Figs 2-4, typically of greater section than each passage 8a, 8b giving access to an interior of valves V1, V2 actuated in a calibration mode.

### Exemplary embodiments for the valves

The section of conduit SC can provide a configuration with the middle segment SC2 and the branches/valve bodies 10a, 10b serving for an active flow of a fluid/flowable media different from the biopharmaceutical fluid F. A longitudinal hollow body/wall body 8, rigid and forming all or part of the wall W, is included in the section of conduit to form the main piece with a pair of valve bodies, also called valve housings 10a, 10b.
The body 8, possibly shaped as a shell or tube to delimit an interior volume included in the lumen, can delimit each radial passage 8a, 8b, at the two hollow valve housings 10a, 10b.

The body 8 is constructed to allow integration, in the assembly 1, of an inlet port 11 and an outlet port 12, each connectable to a portion of a calibration path that includes or forms a calibration line 15. The inlet port 11, provided distal from the middle segment SC2, at a radial distance, can be included in the inlet valve V1, while the outlet port 12, also provided distal from the middle segment SC2, can be included in the outlet valve V2. The inlet port is configured for fluid connection with a calibration line 15, so that in open state of the inlet valve, the lumen 2 can be feed with a solution/media coming from the calibration line: a calibration mode can be actuated by exposing the sensing member sm of a calibratable sensor to such different fluid (caller hereafter calibration process media).

The inlet valve V1 can include a valve member 111, VM that is the movable part guided in the valve housing 10a forming the branching JW at a junction with the middle segment SC2 carrying the mounting structure 5. This valve member 111, VM is a first valve member that can close/seal the first passage 8a, as illustrated for instance ion the left in Fig. 4. A first sealing element J1 can be included in the closing part of the valve member 111, VM to contact the inner surface/seat provided in the valve housing 10a, here adjacent to the lumen 2.
In some options, when an inner channel 17 is delimited by the valve member 111, such channel 17 is displaced with the valve member to be plunged into or facing the lumen 2. Like in the example of Fig. 4, at least the first valve member 111 may be provided with a plunger, shaped as a narrow shell. The first valve member 111 has an insertable part, movable perpendicular to the longitudinal axis A, to be displaced inside the corresponding valve housing 10a.

Referring to Figs 4 and 5A-5B, the first valve member 111 also has an external actuation portion, protruding radially outside an annular end of the valve housing 10a. The length of the valve housing 10a can be inferior to length of the mounting structure 5 and/or length of any of the end section segments SC1, SC2. In this option, the first valve member 111 is guided in the valve housing by a cam, a helical guide or similar relief to allow a further insertion of the valve member 111 when rotated (arrow in Fig. 5B illustrates rotation as allowed by such valve, here around axis Y that is perpendicular to the longitudinal axis A), starting from the closed position. The external actuation portion is provided opposite from one or more access passages O11, which form mouth(s) provided:
- at the proximal end of the channel 17 (close to the longitudinal axis A), and
- for allowing communication between the calibration line (Fig. 1) or suitable path, coupled to the inlet valve (V1) via the inlet port 11, and the measuring area (location of a sensing member sm) formed in the lumen 2.

The outlet valve V2 can include a valve member 112 that is the movable part guided in the valve housing 10b forming another branching JW at a junction with the middle segment SC2 carrying the mounting structure 5. The features provided for the structure and movement of the valve member 112, forming the second valve member in the monitoring assembly 1, can be the same as for the first valve member 111. A helicoidal guiding can be provided.
For each valve, the valve member 111, 112 thus can extend between a first end part forming an insert member inserted in the valve housing 10a, 10b and a second end part, typically forming a fluid outer connecting end that opens (via passages O11, 012, respectively) along a direction perpendicular to the longitudinal axis A. The fluid outer connecting end can be chosen amongst a barbed nozzle, a Luer connecting part LC and a bayonet connection part or comparable quick-coupler.

The valve housing 10a, 10b can be shorter than each channel 17, 18: each elongated hollow channel preferably extends straight or rectilinearly between the opening (port inlet 11 or port outlet 112) at the fluid outer connecting end and a closed end that is:
- adjacent to a plugging or closing part of the valve member 111, 112;
- provided with at least one transverse passage O11, O12 directly communicating with the lumen 2 in open state of the valve.

Now referring to Figs 5A-5B, at least one of the valve housings 10a, 10b can have fixation means FM adapted to cooperate with complementary fixation means (10p, 10r) provided on the valve members 111, 112, to obtain a bayonet connection or similar connection actuated by a rotation to place the closing part with the first sealing element J1 onto an annular seat of the valve housing 10a, 10b. The annular seat is adjacent to the lumen 2 or the branching JW where the valve housing connects to the outer tubular face of the wall W.

The valve member 111, 112 can comprise one or more tabs 10p, each resiliently deformable, for instance two tabs that are extending from the flange portion 10f to a free end. Like options shown in Figs 5A-5B, the tab(s) 10p can extend parallel to the channel 117, 118 (parallel to the valve housing 10a, 10b) and carry a pin or latching member 10r. One or two external grooves or slots f2, engaged by the latching member 10r, can be formed on the valve housing 10a, 10b, possibly with a helical geometry to allow converting rotation at the actuation portion into a insertion to place the closing part on the seat region to seal the corresponding passage 8a or 8b.
Here, each tab 10p is extending outside the valve housing 10a, 10b with the pin or latching member 10 protruding from the tab, toward the valve housing external face to be slidably mounted (in corresponding external groove or slot f2). Figs 4 and 5B illustrate a mounted state for a valve member 111 with each tab 10p allowing a retaining effect when the valve V1 or V2 is in open state.

In options with a bayonet connection, a slot end can be provided with a stop, engaged by the pin or latching member 10 for preventing the valve member 111, 112 to be moved to far outside. Each valve housing 10a, 10b may be provided with an end detent member that is engaged with the pin, relief or latching member 10r, to prevent a reverse displacement of the valve member and keep the open position, while still allowing an unlocking action (due to deformability of the tab 10p, typically). The tab 10p and/or the detent member can be resiliently deformable to allow a removable engagement or snap-action of the latching member, for instance when reaching a groove part G (possibly at an end of a helical slot or recess). At such position with engagement by the detent member 10r, in the groove part G, a pulling action exerted on actuation flange portion 10p cannot drive the valve member in rear direction to open the valve V1, V2, due to a stop action: the groove part 10p is thus locally delimited by a stop. End of the stroke may also be controlled, when reaching the closed position, by at least one of:
- a abutment contact of the flange portion 10p against the valve housing 10a or 10b,
- and an abutment contact at the groove part G, against the groove end delimitation.

In some embodiments, the external actuation portion can have a flange portion 10f (Figs 4, 5A), possibly forming two opposite ears that help for actuating rotation. When starting from a first position, with the flange portion 10f distant/spaced from the valve housing 10a, the guide can induce an insertion so that rotation, for instance of 90°, can end with the flange portion adjacent or in contact with the valve housing 10a. Same structure can be provided for the second valve member 112 l the outlet valve V2, with access passage(s) 012 moved radially inward like access passage(s) O11, to follow the plunger part with the sealing element J1 (elastomer O-ring or the like) that can be located inside the lumen 2. For instance, the closing parts in the valves members 111, 112 can be intersected by the longitudinal axis A, in the open position of the inlet valve V1 and the outlet valve V2.

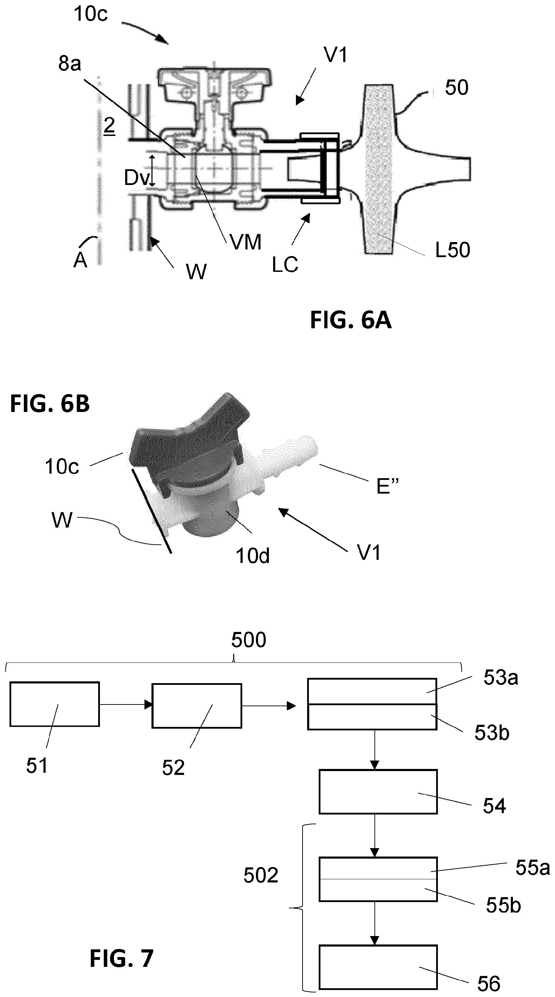

More generally, the valve member 111, 112, VM can be rotated directly by manually actuating a graspable member. For manual operation of the inlet valve V1 and/or the outlet valve V2, the valve member 111, 112, VM can be provided with a flange portion 10f or gripping actuator 10c at the external actuation portion, as well apparent in Figs 4-6B.
While the valve members 111, 112 in options of Figs 4 and 5A-5B have each an end E, E' constructed as fluid coupler, typically nozzle-shaped to form the inlet and outlet valves V1, V2, other options can provide a coupling end E" (also a hose barb end, Luer coupler or the like) that is included in the main plastic piece forming the wall W, as in the non -limiting example of Figs 6A-6B. In such case, the valve body/housing 10d may form a guide for a rotation of the valve member VM without any sliding insertion of the valve member VM.

The inlet valve V1 and the outlet valve V2 can have a compact structure, each with a channel for the calibration process media and any other liquid (rinsing solution in particular) that has an inner diameter Dv. As apparent for instance in Figs 4 and 5A, inner diameter Dv is significantly/strictly inferior to the diameter D2 of the lumen 2. The inner diameter Dv can be the interior diameter of the valve member channel 17, 18, which can be an elongated channel (for instance perpendicular to the longitudinal axis A) inside the movable member 111, 112, VM of the valve V1, V2. In variants (as in Fig. 6A-6B), the inner diameter Dv can be provided in a valve housing 10d that is stationary/integrally formed with the wall W. In preferred embodiments, the following relationship can be satisfied: 0.3 <Dv / D2 < 0.7
Whatever the precise structure of the valve member and the guiding parts, the channel characterizing size, typically inner diameter Dv, can be kept small.

Besides, length d4 of the non-overlapped area of the section of conduit (area not dedicated to connection of the hoses T1, T2), as measured between the two flanges or stopping portions 4s (Fig. 4), can be lower than 110 mm and/or comprised in range of 50 mm to 100 or 200 mm. In some options, total of plastic weight present in in the empty section of conduit SC, including the sensor mount(s) 5a, 5b and as considered as a rigid block (extending as far as the opposite ends/members 4a, 4b) without any flexible hose, can optionally be lower than 80 g when the plastic of the section of conduit SC is a molded thermoplastic.

### Exemplary structure for sealing a valve member

When having a valve member 111, 112 that is displaceable radially toward the lumen 2, two sealing elements J1, J2 can be displaced together with the valve member J1, J2. In such options, each movable valve member 111; 112 may be elongated and/or extend along an extension direction D transverse to the lumen 2. Referring to Fig. 4, each valve V1, V2 may typically comprise:
- the first sealing element J1, provided on the movable valve member 111, 112 at the closing part thereof, away from the actuation portion; and
- the second sealing element J2 secured to the movable valve member 111 ; 112 and having an annular shape to provide an annular sliding contact, onto valve hosing inner face, to allow an interior space ZV of the valve V1, V2 to be separated from an outside of the monitoring assembly 1.
The interior space ZV can have an extension size d2, corresponding to the spacing between the sealing elements J1 and J2. This size d2 is lower than length d1 (Fig. 4) of each valve housing 10a, 10b, and possibly lower than 20 or 24 mm, so that the sealing element J2 can remain in contact with the valve housing and the length of the valve housing can be kept relatively small.

In the closed position as apparent on the left in Fig. 4, the interior space ZV can extend between the two sealing elements J1, J2, without communicating with the lumen 2 due to sealing contact performed by the sealing element J1. In other words in closed state of the valve, the closing part of the valve member 111, 112 can be interposed between the intermediate area ZV and the lumen 2, with the first sealing element J1 (on the closing part) being movable radially inward, starting from a seating position, in which it seats on the valve housing inner face to isolate the lumen 2 from any area/intermediate area still in fluid communication with the calibration line 15 when coupled to the inlet port 11.

In open position, the sealing element J1 follows the closing part and the second sealing element J2 remains in annular contact with valve housing inner face. Accordingly, the valves V1, V2 can be integrated in a compact monitoring assembly 1, while ensuring aseptic connection of each valve member 111, 112 with ease of handling. Sealing contact on the seating region SR to close each valve V1, V2 may optionally be obtained by having the closing part flush with middle segment inner face.

In preferred options, the valves V1, V2 are operated manually, for instance with a rotative actuation of an actuating flange portion 10f, outer shell or actuating lever (lever spatially segregated/radially spaced from the wall W delimiting the lumen 2). In some variants, one or two of the valves V1, V2 could be automated, possibly with a predetermined synchronization. In use, the assembly 1 may be provided downstream a pump 16 (Fig. 3), which is supplying the fluid F in the lumen 2 via the first end of the assembly 1, which is typically the end formed by the first fluid connecting member 4a.

### Embodiments for the sensors

In preferred options, a pH sensor is integrated in the monitoring assembly 1, with a mounting on the mounting structure 5, using selectively a given sensor mount 5a or 5b. Another sensor may also be provided, for instance including a probe or detection means for dissolved oxygen (DO), conductivity, pressure, flow rate, temperature, turbidity, viscosity or analog physical or chemical property.
A sensor 3, 6 can be communicatively coupled to a control unit, while having its sensing member sm possibly provided at a free end, exposed to the fluid circulating in the lumen 2. The sensor or at least one of the sensors can be configured to measure process properties/variables of the process fluid and send control signals comprising the measured process properties/variables to the control unit.

In some embodiments, the disclosure is directed to single use. A sensor 3 or each sensor 3, 6 may be comprised in or secured to the section of conduit SC, to form a single-use unit, which may be disposed, e.g. after the bioprocess is completed.
The sensor(s) can be adapted for multi calibration.
When having two sensor mounts 5a, 5b, the two sensors may be disposed parallel, close to each other, with an interspace not exceeding 15 or 24 mm for instance. They may extend through:
- a common opening delimited by an annular frame of the section of conduit SC,
- and a corresponding sensor mount 5a, 5b provided in a cover plate that seals the common opening/side opening.

The monitoring assembly 1, as illustrated for instance in Figs 1-4, can directly carry a filter 50 that is included in the calibration line 15, with the filter 50 forming the coupling end of the calibration line 15. The filter 50, provided with a layer of filtering media L50 inside a filter housing, can be used in one or more feeding steps to feed a conditioning media to be used in a calibration process (calibration process media) or a cleaning/rinsing media. A coupling connection, for instance Luer connection or a quick coupling can be realized to prevent including any flexible hose between the filtering media 50 and the lumen 2. This allows shortening the calibration path.

The monitoring assembly can comprise the calibration line 15 and possibly the two switching parts 43, 45, which are combinable with the first valve V1 and the second valve V2 to switch between a monitoring mode and a calibration mode. Referring to Fig. 3, the monitoring assembly 1 comprises, in the calibration line 15, reference means 40, 48 and a filter 50 to allow a process media (possibly being WFI), prepared in advance, to be supplied and filtered in the calibration line 15 (upstream the lumen 2) so that the flow coming from the calibration line 15 is a flow of the filtered process media. Water for injection WFI typically constitutes a fluid for some transition steps, for instance at least before setting or coming back to a monitoring mode. A reference standard solution 40 can be circulated and an already calibrated sensor (reference sensor 48) can be involved in the calibration mode. Pumping by the pump 46 provided in the calibration line 15 is of interest to have operations quickly performed, without using a pump that is provided beyond one of the flexible hoses 11, 12 of the main path used of the monitoring mode.

The calibration line 15 can optionally be sanitized with a suitable fluid 42, possibly a sterilizing fluid. A selection device 38 can receive each fluid coming from the different reservoirs or sources, possibly according to a predetermined sequence in each calibration step. Depending on the use cases, any suitable type of fluid may be supplied to the lumen 2 via the calibration line 15. Figure 3 shows a convenient setup with a selector device 38, such as a valve component, for selectively connecting reservoirs of a reference standard solution 40, a sterilization or sanitation fluid 42 and water for injection (WFI) 44 to the calibration line 15. The filter 50 can include a disposable, pre-sterilized, ready-to-use membrane filter capsule for highest convenience.

The monitoring assembly 1 is adapted to be set in:
- a first configuration to define a first flow path (main path), in which the two switching parts 43, 45 are open to allow a continuous circulation along a rectilinear main channel formed all a whole length of the section of conduit SC, while the inlet port 11 for communication with the calibration line 15 is remaining closed by closing the inlet valve V1, whereby the monitoring mode is obtained; and
- a second configuration to define a second flow path, in which the two switching parts 43, 45 are closed, while the inlet valve V1 and the outlet valve V2 are each in an open state to have a flow coming from the calibration line 15 circulating successively through the inlet port 11, through the lumen 2 and through the outlet port 12.

### Exemplary steps involved in calibration mode

The present assembly can be used with more than two sensors and/or with a pH sensor device that is calibratable (possibly also recalibratable, i.e. calibratable twice or more than twice). It is understood that none of the setups allowed by the monitoring assembly 1 requires a bypass line for bypassing the sensor. In particular, the flow of the biopharmaceutical fluid F through the main path can be interrupted by the first clamp/switching part 43 upstream of the sensor(s) 3, 6. At the same time, the inlet valve V1 can establish a calibration flow path for the calibration procedure, including the calibration line 15, the channel 17 and the lumen 2 leading through the sensor 3, 6 to be calibrated. The waste receiving part V60 or similar volume of discharge line 20 completing the calibration line 15 can receive the calibration process media leaving the lumen 2 via the outlet port 12.

The fluid monitoring assembly 1 can be subjected to a method for in-situ calibration, after ensuring the switching parts 43, 45 have been set to stop fluid F of the main path to flow through the monitoring assembly 1. This is a preliminary step 51 performed by actuating, typically manually, the pair of switching parts 43, 45, possibly simply designed as clamps, to have the lumen 2 inside the section of conduit SC (where measuring area is formed) isolated from other portions of the main flow path where the given biopharmaceutical product F can flow. A distributed switching, away from the middle segment SC2, is typically allowed to close:
- the first fluidic connection member 4a or a hose T1 coupled thereto, and
- the second fluidic connection member 4b or a hose coupled T2 thereto.
In some alternative options, all or part of the switching parts 43, 45 can be automated, for instance with use of two automated valves.

In embodiments, the fluid monitoring assembly 1 can be provided preassembled and it can directly be used for an initial calibration, which implies opening the valves V1, V2, while maintaining the switching parts 43, 45 in closed state.

Now referring to Figs 3 and 7, the preliminary actuation step 52 (optional step, not required when all the switching parts are already closed) and other steps 53a, 53b can be performed in a preparation phase 500 allowing proper configuration of the monitoring assembly 1 to be obtained, before using solution(s) from the calibration line 15. The actuation step 52 can be considered as a step of stopping the monitoring mode, when calibration must be performed after a first use of the section of conduit SC with the sensor(s) already delivering detected measures.
The assembling 51 of the calibration line 15 can be performed in advance, for instance to provide a single-use preassembled assembly (sterile assembly, preferably). The assembling 51 can be performed only once to keep sterility: no separation of disconnected parts occurs in any operating mode of the assembly. In contrast, steps involving the valves V1, V2 and switching parts 43, 45 can be repeated.
Accordingly, the assembling step 51 has been here illustrated as a step performed during the preparation phase 500, typically before the preliminary step 52 to isolate the lumen 2 if needed. In a variant, such assembling 51 could be finalized after the actuation step 52.
The assembling 51 can allow components such as illustrated in Fig. 3 to be assembled in the calibration line 15, with a coupling performed to make the inlet port 11 connected fluidically to the calibration line 15 with circulation allowed by a pump 46 provided outside the section of conduit SC. The coupling of the calibration line 15 to the inlet port 11 can directly involve the filter 50, which is possibly mounted downstream one or more reference sensor(s) 48. The discharge line 20 is also connected, possibly with the valve V2 closed, thanks to the assembling 51.

In the preparation phase 500 to prepare calibration of at least one sensor 3, 6, fluid connection is established at an end of the calibration line 15, to have the fluid connection to a reference source or a reference standard solution 40 related to a physical or chemical property to be measured or detected by the sensor 3, 6. The solution 40, which can directly form the calibration process media, is provided for the purpose of calibrating a specific sensor, for instance a pH sensor integrated in a corresponding sensor mount 5a, 5b of the monitoring assembly 1. The calibration line 15 can be assembled by having such fluid connection (first fluid connection) with the reference source and then a second fluid connection with the measuring area established via the opening of the inlet valve V1 at a later step. As illustrated in Fig. 7, the preparation phase 500 may be ended by:
- functionally connecting 53a the calibration line 15 to the inlet port 11, with fluid communication established by setting the inlet valve V1 in open state; and
- functionally connecting 53b the discharge line 20 to the outlet port 12, with fluid communication established by setting the outlet valve V2 in open state.

Whatever the precise order for the steps, each valve member 111, 112, VM can possibly be moved when carrying an end hose of one of these lines 15, 20, being actuated by a limited rotation, typically inferior or equal to 90°. In embodiments of Figs 4 and 5A-5B, the inlet port 11 can be an end part of the first valve member 111 and the outlet port 12 can be an end part of the second valve member V2, with each actuation portion 10f interposed between the rigid block forming the section of conduit SC and a line end component that locally surrounds the corresponding port (inlet port 11 or outlet port 12).

With or without a reference sensor 48 (either single-use or reusable), which is preferably of the same type as the sensor 3, 6 to be calibrated, the calibration line 15 can firstly feed the lumen 2 by the calibration process media, including a reference standard solution 40, typically with at least one exactly known property (pH, dissolved oxygen (DO), conductivity, turbidity, viscosity etc.) for this solution 40. The reference standard solution 40 can form a calibration process media once filtered by the filter 50 that can be arranged adjacent the inlet valve V1.

While a single filtering stage using a filtering layer or media L50, separating at least solid particles for instance, has been illustrated to have the solution 40 filtered before reaching the measuring area in the lumen 2, any other suitable separation stage can be used at near a junction between the calibration line 15 and the section of conduit SC, typically upstream the inlet valve V1.
A filtering step can be systematically performed during any circulation phase using a liquid solution/fluid from the calibration line 15 to the discharge line 20 via the lumen 2. The feeding 55a to provide the calibration process media, in the measuring area, can be initiated by the pump 46. Referring to Fig. 7, such feeding may be preceded by a rinsing with a flowing of a rinsing solution 44 through the measuring region/area to place the sensing member sm of the sensor 3, 6 in a flow of this rinsing solution 44 (WFI for instance) admitted via the inlet valve V1 and discharged via the outlet valve V2. Other solution(s) 42 can be involved, for instance a sterilization or sanitation solution, before the rinsing for instance.

The calibration mode then includes a measuring period, where one or more measured values detected by the sensor 3, 6 are retrieved. During the calibration, it is understood, different fluids can circulate through the lumen 2 until circulation phase 502 using the calibration line 15 is effective, to have proper calibration data on the one hand, and suitable conditions before switching to the monitoring mode with circulation of the biopharmaceutical product F. The rinsing can allow a pH sensor (forming a sensor placed in the mounting structure 5) to be wet up: few mL to several Liters of WFI in some examples, buffer or other biopharmaceutical fluid. The electrode or similar sensing member can be exposed to the different fluids until it is operational. In some options, it may imply a flushing out of any kind of protection or medium that is typically arranged around the sensing member sm.

The monitoring assembly 1 can be submitted to a first calibration. When a pH sensor is part of the measuring part of the monitoring assembly 1, such sensor can generally be supplied pre-calibrated but additional calibration points may be necessary. More generally, the reference standard solution 40 or a first calibration solution can be pushed through the measuring area, reaching the sensor 3, 6. And then the calibration line 15 is rinsed with WFI, flowing to the discharge line 20. This operation can be repeated if a second point a calibration is needed.

Now referring to Figs 3 and 8, use of the calibration line 15 with the circulation phase 502 can be efficient with simple manual operations involving the selection device 38, the valves V1, V2, the switching parts 43, 45 and the suitable pumping component(s), for instance two pumps 16, 46. Regarding the rinsing steps 54, 54', they may be implemented during the circulation phase 502 using the calibration line 15 and the discharge line 20: for instance, a rinsing of the sensor 3, 6 is performed before the calibration step with retrieval 56 of suitable data/values. Then, another rinsing 54', to place each sensing member sm of sensor(s) 3, 6 in the flow of the rinsing solution, is performed after the calibration step.

By a closure of the inlet and outlet valves V1, V2 at the closure step 57 (so that the lines 15 and 20 are separated from the lumen 2) and thanks to a suitable reverse actuation 58, which is the opposite of actuation 52, to allow circulation form the hose T1 to the hose T2 via the lumen 2, the main fluid path can be reactived/open. The different sections SC1, SC2 and SC3 are thus guiding the biopharmaceutical fluid F with at least one sensor 3 (Fig. 8) providing the measures in the monitoring mode.

The monitoring in process can be realized, since the calibration path (with lines 15, 20) is closed, and the bioprocess is running with typically continuous measurement, for instance including continuous PH measurement with the settings. No mechanical disconnection is required to enter such phase, typically with none of the first fluidic connection member 4a) and the second fluidic connection member 4b carrying any switching means. As a result, the section of conduit SC can be of simple conception, with easy integration of each sensor 3, 6 and valve member 111, 112, VM.

As illustrated in Fig 8 for instance, the monitoring assembly 1 can easily allow recalibration. The first calibration can be reiterated/repeated any time it is needed, after stopping the flow path with step 52, here after step 58 with a phase of monitoring. A proper actuation 53' allows the calibration line 15 and the discharge line 20 to be connected again to the lumen 2 via the actuation of the respective valve members 111, 112, VM.
Then, the circulation steps of the circulation phase 502 can be repeated; possibly starting with a rinsing 54 of the lines 15, 20 with WFI or similar rinsing solution.

The monitoring assembly 1 can have two sensors 3, 6 and two main pieces: a cover plate 36 of the mounting structure 5 and the section of conduit SC, to minimize the assembling operations and amount of plastic parts. Use of a same common plastic piece to carry the sensors or suitable measuring instruments typically on a same side, via the mounts 5a, 5b, can be a preferred option. In some variants, the sensors can be distributed in different angular sectors, preferably with the sensing members being in a same region of the middle segment SC2 to have the operative flow length, in the lumen 2, reduced and lowered (for instance lowered of at least 40%) as compared to the assembly length as defined at the opposite axial ends in the connecting members 4a, 4b.

Besides, it is understood that any suitable structure can be provided to have a closing of the main path flow using the full length of the assembly 1, with clamps, pinching members, fastening members or any suitable switching parts.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

## Claims

1. A flowable biopharmaceutical product monitoring assembly (1), comprising:
- a segment of conduit (SC) comprising a wall (W) defining a lumen (2) through which the flowable biopharmaceutical product passes, the segment having a longitudinal axis (A) and extending longitudinally between a first fluidic connection member (4a) for attachment to a flexible hose (T1) and a second fluidic connection member (4b) for attachment to another flexible hose (T2), the segment;
- at least one sensor mount, integrally formed with the wall (W) of the segment of conduit, comprising a sensor mount (5a) extending generally transverse with respect to the longitudinal axis of the segment of conduit (SC), the sensor mount (5a) including an aperture (5c) defining an inner surface extending through the sensor mount to the lumen (2);
- a sensor (3; 6) secured, preferably removably, within the sensor mount (5a), so that the sensor (3; 6) is an integrated sensor for measuring or detecting a physical or chemical property, preferably by measuring pH, at a first measurement location in the lumen (2);
- an inlet valve (V1) and an outlet valve (V2), each formed on a side of the wall (W);
- a longitudinal wall body (8), rigid and forming all or part of the wall (W), preferably shaped as a shell to delimit an interior volume included in the lumen (2), the longitudinal wall body (8) comprising two hollow valve housings (10a, 10b) and allowing integration, in said assembly (1), of an inlet port (11) included in the inlet valve (V1) and of an outlet port (12) included in the outlet valve (V2), the inlet port (11) being configured for fluid connection with a calibration line (15);
wherein the at least one sensor mount is provided between two spaced passages through the wall that consist of:
- a first passage (8a) for radial fluid communication between an inlet port opening and the lumen (2), the inlet port opening being provided in the inlet valve (V1) and the first passage (8a) being closable by a first movable member (111; VM) of the inlet valve (V1); and
- a second passage (8b) for radial fluid communication between the lumen (2) and an outlet port opening provided in the outlet valve (V2), the second passage (8b) being closable by a second movable member (112; VM) of the outlet valve (V2) with the outlet port opening provided beyond a closing part of the second valve member (112; VM) that is movable in said body (8), the closing part being displaceable in a hollow adjacent to the lumen (2), wherein the inlet valve (V1) and the outlet valve (V2), each adjacent to the wall (W), respectively allow the calibration line (15) and a discharge line (20) to be connected to the lumen, in a calibration mode with the first passage (8a) released due to open configuration of the inlet valve (V1).

2. The monitoring assembly according to claim 1, wherein:
- the inlet valve (V1) has a movable valve member (111) delimiting at least one fluid channel (17) of the inlet port (11) and provided with an actuatable portion (10p), preferably a rotating flange, that carries or includes an nozzle (E) forming an end of the at least fluid channel (17); and
- the outlet valve (V2) has a movable valve member (111; 112) delimiting at least one fluid channel (18) of the outlet port (12) and provided with an actuatable portion (10p), preferably a rotating flange, that carries or includes another nozzle (E') forming an end of the at least fluid channel (17).

3. The monitoring assembly according to claim 1 or 2, wherein each valve amongst the inlet valve (11) and the outlet valve (12) comprises:
- a valve housing delimited by a tubular wall section that is perpendicular to the longitudinal axis (A) and opens into the lumen (2); and
- a valve member (111; 112) that is displaced radially inward to move from an open position distal from the longitudinal axis to a closed position proximal to the longitudinal axis, preferably with the valve member (111; 112) protruding inside the lumen (2).

4. The monitoring assembly according to claim 3, wherein for each valve, the valve member (111; 112) extends between a first end part forming an insert member inserted in the valve housing and a second end part, forming a fluid outer connecting end that opens, at an opening, along a direction perpendicular to the longitudinal axis, chosen amongst a barbed nozzle, a Luer lock connecting part and bayonet connection part,
and wherein the elongated hollow channel extends, straight or rectilinearly, between the opening at the fluid outer connecting end and a closed end that is:
- adjacent to a plugging or closing part of the valve member;
- provided with at least one transverse passage directly communicating with the lumen in open state of the valve.

5. The monitoring assembly according to claim 1, 2 or 3, wherein at least one valve amongst the inlet valve (11) and the outlet valve (12) is actuatable by a rotating action, using a bayonet connection for securing the valve member (111; 112), which includes an elongated hollow channel (17; 18), to a valve housing (10a; 10b) that is preferably integrally formed of molded plastic material with a rigid piece of the section of conduit (SC).

6. The monitoring assembly according to any one of the preceding claims, wherein the sensor (3; 6) has an elongated body (B3) terminating at one end thereof in a sensing member (sm), the elongated body (B3) having a flange (C3) on a portion thereof that rests on or within the sensor mount (5a) when secured within the sensor mount (5a).

7. The monitoring assembly according to any one of the preceding claims, wherein the sensor mount is a first sensor mount (5a) and the sensor is a first sensor (3) provided with a first sensing member configured to be in contact with fluid, forming the biopharmaceutical product, passing through the lumen (2),
wherein the monitoring assembly (1) comprises:
- a second sensor mount (5b) integrally formed with the wall (W) of the segment of conduit and extending generally transverse with respect to the longitudinal axis (A) of the segment of conduit (SC), the second sensor mount including an aperture (5d) defining an inner surface extending through the second sensor mount to the lumen (2) of the segment of conduit; AND
- a second sensor (6), which is secured, preferably removably, within the second sensor mount (5b), the second sensor (6) being provided with a second sensing member configured to be in contact with the fluid passing through the lumen, so that the second sensor (6) is another integrated sensor for measuring or detecting a physical or chemical property at a second measurement location in said lumen.

8. The monitoring assembly according to any one of the preceding claims, wherein each valve amongst the inlet valve (V1) and the outlet valve (V2) comprises:
- a movable valve member (111; 112) having an extension direction (D) transverse to the lumen (2);
- a first sealing element (J1) provided on the movable valve member (111; 112), configured to be in sealing contact on a seating region (SR) formed by the section of conduit (SC) to close the valve; and
- a second sealing element (J2) provided on the movable valve member (111; 112) and having an annular shape, the second sealing element (J2) following displacement of the movable valve member and separating an interior space (ZV) of the valve from an outside of the monitoring assembly (1),
wherein the first sealing element (J1) and the second sealing element (J2) share a common central axis and/or are distributed, with a spacing, along the extension direction (D) of the movable valve member (111; 112).

9. The monitoring assembly according to any one of the preceding claims, formed as a single-use assembly comprising a first hose barb connector and a second hose barb connector that are:
- inseparable, preferably made of same material in a same molded piece;
- facing opposite directions, while extending parallel to the longitudinal axis (A), the longitudinal axis (A) preferably being a symmetry axis for the first hose barb connector and the second hose barb connector, which constitute the first fluidic connection member (4a) and the second fluidic connection member (4a);
wherein the wall (W) is assembled from:
- a first piece forming the body (8), including the first hose barb connector and the second hose barb connector, and
- a second piece that is a cover plate (36) having a top surface and including the at least one sensor mount formed on the top surface side,
and wherein the wall (W) is provided with two opposite side recesses (R1, R2) to form a handle part facilitating grasping of the monitoring assembly (1) underneath the cover plate (36).

10. The monitoring assembly according to any one of the preceding claims, comprising the calibration line (15) and two switching parts (43, 45) combinable with the inlet valve (V1) and the outlet valve (V2) to switch between a monitoring mode and a calibration mode, the monitoring assembly (1) being adapted to be set in:
- a first configuration to define a first flow path, in which the two switching parts (43, 45) are open to allow a continuous circulation along a rectilinear main channel formed all a whole length of the section of conduit (SC), while the inlet port (11) for communication with the calibration line (15) and the outlet port (12) remain both closed by closing the inlet valve (V1) and the outlet valve (V2), whereby the monitoring mode is obtained; and
- a second configuration to define a second flow path, in which the two switching parts (43, 45) are closed while the inlet valve (V1) and the outlet valve (V2) are each in an open state to have a flow coming from the calibration line (15) circulating successively through the inlet port (11), through the lumen (2) and through the outlet port (12);
wherein the monitoring assembly (1) comprises, in the calibration line (15), reference means (40, 48) and a filter (50) to allow a process media, prepared in advance, to be supplied and filtered in the calibration line (15) upstream the lumen (2) so that the flow coming from the calibration line (15) is a flow of the filtered process media.

11. A method for calibrating a sensor (3; 6) in a measuring area where a monitoring is also performed for a given biopharmaceutical product, wherein the fluid monitoring assembly (1) according to any one of claims 1-10 is provided so that the measuring area is located in an interior volume of the segment of conduit (SC), between the inlet valve (V1) and outlet valve (V2) on the one hand, and between the first fluidic connection member (4a) and the second fluidic connection member (4b) on the other hand,
wherein the method comprises, in a preparation phase (500):
- actuating (52) switching parts (43, 45) to have the lumen (2) isolated from other portions of a main flow path where the given biopharmaceutical product (F) can flow, the lumen (2) being delimited by an inner face of the segment (SC) that is constructed as a rigid part;
- functionally connecting (53a) a calibration line (15) to the inlet port (11) by opening the inlet valve (V1);
- functionally connecting (53b) a discharge line (20) to the outlet port (12) by opening the outlet valve (V2);
and wherein the method then comprises a calibration of the sensor (3; 6) mounted between the inlet valve (V1) and the outlet valve (V2), which is performed with flowing of a calibration process media in a circulation phase (502), the circulation phase (502) comprising:
- feeding (55a) the measuring area in the calibration process media via the inlet valve (V1) open, while the sensor (3; 6) arranged in the measuring area is in contact with the calibration process media;
- allowing the calibration process media to be discharged (55b) from the lumen (2) to the discharge line (20) via the outlet port (12) to have a preferably discontinuous circulation of the calibration process media through the measuring area; and
- retrieving (56) a measured value detected by the sensor (3; 6).

12. The method according to claim 11, wherein the switching parts (43, 45) are distributed to close the first fluidic connection member (4a) or a hose (T1) coupled thereto, and the second fluidic connection member (4b) or a hose coupled (T2) thereto, respectively, wherein, the method comprises, in the preparation phase (500):
- assembling (51) the calibration line (15) provided with a pump (46) and a filter (50), the calibration line (15) being further provided with at last one amongst a reference sensor (48), a reference source or a reference standard solution (40) related to a physical or chemical property to be measured or detected by the sensor (3; 6);
and wherein the calibration process media is filtered in the calibration line (15) before reaching the measuring area, during said feeding (55a) in the calibration process media, said feeding (55a) using the pump (46).

13. The method according to claim 11 or 12, wherein the sensor (3), which is preferably a single-use sensor, is a first sensor (3) of the monitoring assembly (1), while a second sensor (6) is mounted on and secured to the segment of conduit (SC) that carries the first sensor (3), so that the first sensor (3) and the second sensor (6) are both mounted with a sensing member located within said measuring area, between the inlet valve (V1) and the outlet valve (V2),
and wherein the monitoring is performed by using the first sensor (3) and the second sensor (6) simultaneously, after at least one calibration performed for the first sensor (3), with the given biopharmaceutical product flowing straight through the first fluidic connection member (4a), the lumen (2) and the second fluidic connection member (4b).

14. The method according to claim 11, 12 or 13, comprising, before and/or after the monitoring:
- placing (54, 54') a sensing member of the sensor (3) in a flow of a rinsing solution (44) distinct from the calibration process media (40), the rinsing solution (44) flowing through the measuring area by circulating through the inlet valve (V1) and being discharged via the outlet valve (V2),
wherein the inlet valve (V1) and the outlet valve (V2), spaced from a longitudinal distance (d) inferior or equal to 70 or 80 mm, have each a rigid valve body (10a, 10b) formed integrally with the segment of conduit (SC),
and wherein the sensor (3) is a calibratable pH sensor.

15. The method according to claim 14, wherein calibration of the pH sensor is carried out more than twice, with the outlet port (12) having a hose barb engaged with a hose or female connecting member, which communicates with an interior volume of a waste container or bag (60),
and wherein the monitoring assembly (1) is operating with the inlet valve (11) and the outlet valve (12) open simultaneously during each calibration, while said assembly (1) is maintained in a sealed and sterile configuration, without any mechanical disconnection at the first fluidic connection member (4a) and at the second fluidic connection member (4b).
